# EUROPEAN PATENT APPLICATION

(11) **EP 3 114 981 A1**
(43) Date of publication of application: **11.01.2017**
(21) Application number: 15758041.6
(22) Date of filing: 24.02.2015
(51) Int. Cl.: A61B 1/00

(54) **INSERTION INSTRUMENT**

(30) Priority: 04.03.2014 JP 2014041945
(71) Applicant: Olympus Corporation, Tokyo 192-8507 (JP)
(72) Inventor: KOYAMA Reiji, Tokyo 192-8507 (JP)
(74) Representative: Schicker, Silvia
(86) International application number: PCT/JP2015/055176
(87) International publication number: WO 2015/133330

(57) **Abstract**

By causing a screw receiving portion 20e to protrude from a cord fastening device 150 side of a main frame 20 and by forming the screw receiving portion 20e in a cup-like stepped shape that is recessed from a lower-side chain cover 30 side toward a lower surface side of the main frame 20 by press working or the like of a sheet metal material, a thickness (H-L) of a bottom portion of the cup shape in which a screw hole is formed can be formed to be equal to a plate thickness t of the main frame 20, and a required screw hole depth can be uniformly and accurately secured while maintaining a required minimum plate thickness. Further, a depth L of a concave portion of the screw receiving portion 20e is formed to be a depth such that a distal end portion of a screw 130 to be used does not protrude from the main frame 20, and thus interference between the distal end portion of the screw 130 and the lower-side chain cover 30 can be surely avoided.

## Description

### Technical Field

The present invention relates to an insertion instrument having an insertion portion to be inserted into a subject.

### Background Art

Insertion instruments, for example, endoscopes, that are inserted into a subject have been widely utilized in a medical field and an industrial field in recent years. Such insertion instruments enable the performance of various kinds of observation, treatment and the like by inserting an elongated insertion portion into a subject. A configuration is known in which an insertion portion of an endoscope as such kind of insertion instrument includes a bending portion that is bendable in a plurality of directions, and in which an operator can grasp and operate an operation portion that is connected to the insertion portion to thereby cause a distal end of the insertion portion to perform a bending operation in a desired direction.

In general, a bending portion provided in an insertion portion is configured so as to be bendable in, for example, the four directions of upward, downward, left and right, by connecting a plurality of bending pieces using rivets along a longitudinal axis direction of the insertion portion. Among the plurality of bending pieces, a wire is connected to a bending piece positioned on the distalmost end side, and by winding the wire around a pulley or by connecting a chain to the wire and winding the chain around a sprocket and then rotating the pulley or sprocket to perform an operation to pull the wire, the insertion portion can be caused to perform a bending operation in any of the upward, downward, left and right directions.

As disclosed in Japanese Patent Application Laid-Open Publication No. 9-238895, a mechanism that drives a pulley or a sprocket for performing an operation to pull such kind of wire for bending is assembled in a base fixed inside the operation portion. The base is a frame that serves as a framework of the structure, and in order to reduce the weight of the operation portion, a frame is adopted that is formed by press working of a steel plate from a conventional frame formed by die casting of aluminum.

To achieve a further reduction in the weight of the operation portion, there is a demand to make the plate thickness of the frame that serves as the framework of the structure as thin as possible.

However, since various members are fixed in the frame using screw components, as long as variations exist in the plate thickness of the frame or variations exist in the overall lengths of the screw components themselves, when considering the length of the screw components, the plate thickness of the frame and the fastening length of required screw components, there is a possibility that, depending on the state of such variations, the distal end of a screw component will protrude from the frame.

In such a case, there is a possibility that an area will arise at which a part of the distal end of a screw component interferes with another member that is disposed on an opposite side of the frame.

In this case, although it is also conceivable to use a screw component of a special length and adopt a setting such that the distal end never protrudes from the frame, not only will the use of a special screw component lead to an increase in costs, since an area at which the distal end of the screw interferes is just one part, work must be carried out while making a distinction between the special screw component and screw components of other lengths, which leads to a deterioration in workability as well as complicated management.

In addition, a method can also be considered which, by adjusting the plate thickness of the frame, uses screw components of a standard length that are not a special length. However, in such a case it is difficult to realize the original aim, namely to further reduce the weight of the operation portion by making the plate thickness of the frame as thin as possible.

The present invention has been made in view of the above described circumstances, and an object of the present invention is to provide an insertion instrument that, while achieving a reduction in the weight of a frame which serves as a framework of an operation portion of the insertion instrument, prevents interference between a fixing device which fixes a member to a frame, and another member disposed on an opposite side of the frame.

### Disclosure of Invention

### Means for Solving the Problem

An insertion instrument according to one aspect of the present invention includes: an insertion portion to be inserted into a subject; an operation portion that is connected to the insertion portion and which an operator operates to actuate a distal end of the insertion portion; a plate-like frame that is arranged inside the operation portion and that is fixed to the operation portion; a long member that is arranged inside the operation portion and which actuates the distal end of the insertion portion in accordance with an operation of the operation portion; a cover member which is arranged on the frame and that covers a portion in which the long member travels; a fastening device that holds a member that is arranged inside the operation portion on an opposite side to the cover member of the frame; a fixing device that fixes the fastening device to the frame; and a receiving portion which is formed as a receiving portion for the fixing device in the frame, and which is protruded from a side facing the fastening device of the frame and is formed in a concave shape on a side facing the cover member.

### Brief Description of the Drawings

Fig. 1 is an external view of an endoscope as an insertion instrument;
Fig. 2 is a perspective view of a principal portion of a bending operation mechanism;
Fig. 3 is a cross-sectional view of the area around a spindle of the bending operation mechanism;
Fig. 4 is an explanatory drawing illustrating a fixing structure of a spindle via a connecting plate;
Fig. 5 is an explanatory drawing illustrating a line-of-sight direction at a time of work to fix the spindle shown in Fig. 4;
Fig. 6 is an explanatory drawing illustrating a fixing structure of a spindle of a bending operation mechanism that uses a pulley;
Fig. 7 is an oblique perspective view illustrating a lower surface side of a main frame;
Fig. 8 is an oblique perspective view in which the area around an earth terminal in Fig. 7 is exposed;
Fig. 9 is an explanatory drawing illustrating a connection between a main frame and a cord fastening device;
Fig. 10 is a cross-sectional view along a line X-X in Fig. 9;
Fig. 11 is an enlarged view of an area around a boss portion in Fig. 10;
Fig. 12 is an explanatory drawing illustrating a temporary fixing structure for fixing the cord fastening device to the main frame;
Fig. 13 is an explanatory drawing illustrating another temporary fixing structure for fixing the cord fastening device to the main frame; and
Fig. 14 is an explanatory drawing illustrating a further temporary fixing structure for fixing the cord fastening device to the main frame.

### Best Mode for Carrying Out the Invention

An embodiment of the present invention is described hereunder with reference to the attached drawings. Although the present invention is applied to an insertion instrument having an insertion portion that is inserted into a subject and is bendable, such as an endoscope, a guide tube, various kinds of treatment instruments, and a manipulator, in the embodiment described hereunder an endoscope is described as an example of the insertion instrument.

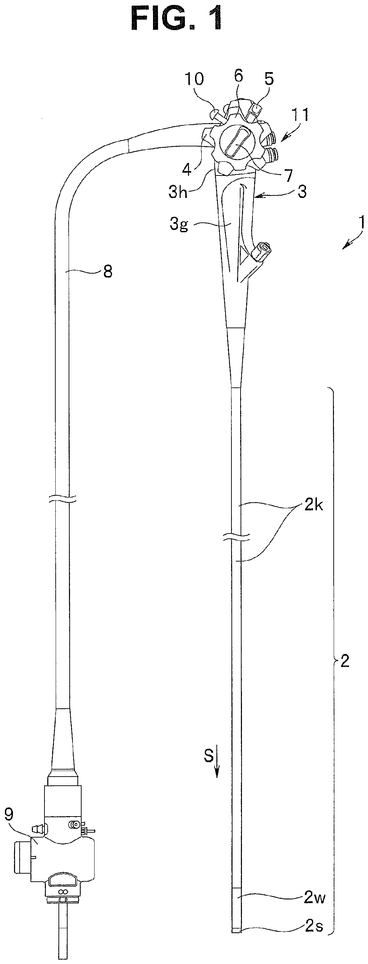

In Fig. 1, reference numeral 1 denotes an endoscope. A principal portion of the endoscope 1 is constituted by including an insertion portion 2 to be inserted into a subject, an operation portion 3 that is connected to a proximal end of the insertion portion 2, a universal cord 8 that is extended from the operation portion 3, and a connector 9 that is provided at an extending end of the universal cord 8. Various kinds of pipe sleeves and various kinds of electric contact points which are not shown in the drawing are provided in the connector 9 of the universal cord 8. External apparatuses such as a video processor and a light source apparatus are electrically connected to the connector 9.

Note that the universal cord 8 is a composite cable through which various electrical cables for signals that are extended from inside the insertion portion 2 via the inside of the operation portion 3, a light guide that guides an illuminating light from a light source apparatus, an air/water feeding tube that is extended from an air/water feeding apparatus and the like are inserted.

A principal portion of the insertion portion 2 is constituted by including: a long flexible tube portion 2k that extends along an insertion direction S of the insertion portion 2; a bending portion 2w which is located at a position that is more frontward in the insertion direction S (hereunder, referred to simply as "frontward") than the flexible tube portion 2k and which is bendable in, for example, the four directions of upward, downward, left and right; and a distal end portion 2s which is located at a position that is more frontward than the bending portion 2w. An unshown image pickup unit that picks up an image inside a subject, and an unshown illumination unit that supplies an illuminating light into a subject and the like are provided inside the distal end portion 2s.

A principal portion of the operation portion 3 is constituted by including: a grasping portion 3g which is grasped by an operator; and an operation portion main body 3h that is connected to a proximal end of the grasping portion 3g and from which the universal cord 8 is extended. Various operation members are provided on the operation portion main body 3h. The operation members include, for example, a bending operation knob 4 for an upward/downward bending operation that causes the bending portion 2w to bend in the upward/downward directions, a bending operation knob 6 for a left/right bending operation that causes the bending portion 2w to bend in the left/right directions, fixing levers 5 and 7 that fix rotational positions of the bending operation knobs 4 and 6, and a zoom lever 10 of an image pickup unit which is provided inside the distal end portion 2s. In addition, switches 11 and the like for remotely operating an external apparatus such as a video processor are also provided on the operation portion main body 3h.

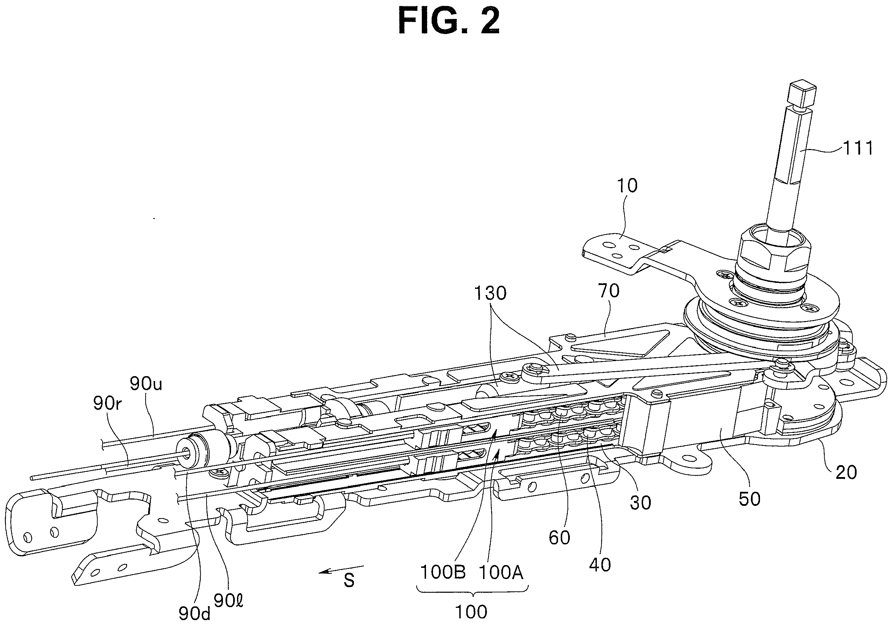

Next, the internal configuration of the operation portion 3 will be described. A bending operation mechanism 100 as illustrated in Fig. 2 is arranged inside the operation portion 3. The bending operation mechanism 100 is a mechanism unit for causing the bending portion 2w at the distal end of the insertion portion 2 to perform a bending operation, in which each member constituting a mechanism portion is fixed to a main frame 20 that is a framework structure. The main frame 20 is formed, for example, by subjecting a metallic material such as aluminum or stainless to sheet-metal working or the like, and is fixed with screws inside the operation portion main body 3h and the grasping portion 3g.

In the present embodiment, the bending operation mechanism 100 is constituted by including a left/right bending operation mechanism 100A for causing the bending portion 2w to perform a bending operation in the left/right directions, and an upward/downward bending operation mechanism 100B for causing the bending portion 2w to perform a bending operation in the upward/downward directions. Note that the left/right bending operation mechanism 100A and the upward/downward bending operation mechanism 100B have approximately the same configuration. Further, reference numeral 130 denotes a link mechanism of the zoom lever 10.

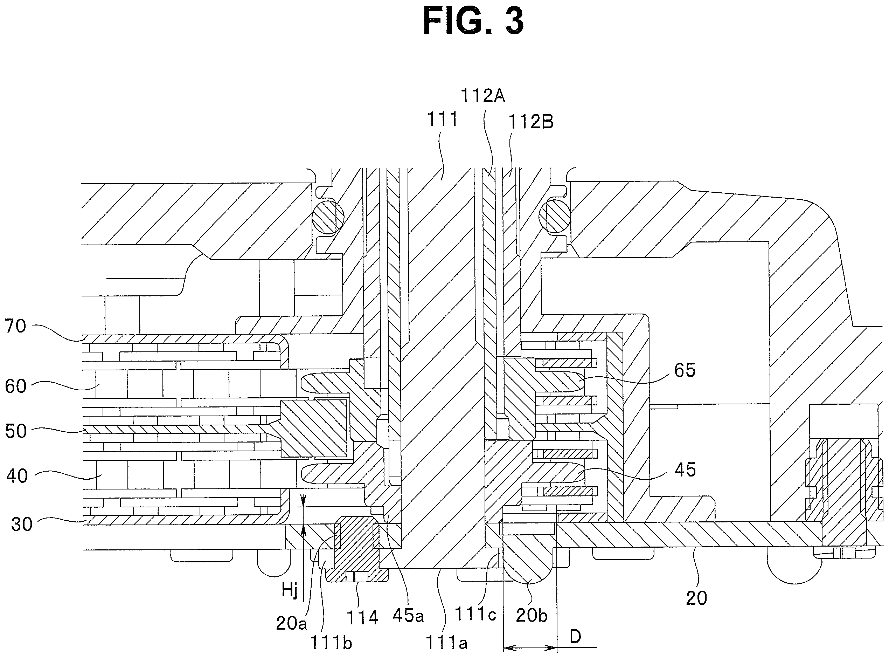

The left/right bending operation mechanism 100A includes a sprocket 45 (see Fig. 3) for left/right bending that is a rotary body that is rotatable with respect to a spindle 111 erected on the main frame 20, and a chain 40 for left/right bending that is a long member that is wound around the sprocket 45, which are disposed sandwiching a plate-like lower-side chain cover 30 on an upper face of the main frame 20. A wire 90r for right-side bending that is extended from the bending portion 2w is connected to a distal end on one side of the chain 40, and a wire 901 for left-side bending that is extended from the bending portion 2w is connected to a distal end on the other side of the chain 40.

A lower-side chain cover 30 is attached to the main frame 20, as a cover member that covers a portion in which the chain 40 travels on the main frame 20.

The sprocket 45 for left/right bending includes a boss portion at which a chain wheel which is a toothed wheel which the chain 40 is wound around is mounted. One portion of an end face of the boss portion protrudes from an opening portion formed in the lower-side chain cover 30. The portion of the end face of the boss portion that protrudes from the lower-side chain cover 30 is disposed so as to contact against the upper face of the main frame 20.

Further, the upward/downward bending operation mechanism 100B includes, on the upper side of the left/right bending operation mechanism 100A, a sprocket 65 (see Fig. 3) for upward/downward bending that is a rotary body that is rotatable with respect to the spindle 111, and a chain 60 for upward/downward bending that is a long member that is wound around the sprocket 65, which are disposed sandwiching a chain separator 50 as a guide member. A wire 90u for upper-side bending that is fixed to a distal end of the bending portion 2w is connected to a distal end on one side of the chain 60, and a wire 90d for lower-side bending that is fixed to a distal end of the bending portion 2w is connected to a distal end on the other side of the chain 60. A plate-like upper-side chain cover 70 is disposed on an upper face of the upward/downward bending operation mechanism 100B.

As shown in Fig. 3, a lower end of the spindle 111 is vertically arranged on the main frame 20, and an upper end thereof passes through the chain separator 50 and the upper-side chain cover 70 and protrudes to outside of the operation portion main body 3h. A tubular shaft body 112A is rotatably disposed on an outer circumferential face of the spindle 111. Further, a tubular shaft body 112B is rotatably disposed on an outer circumferential face of the shaft body 112A.

These two shaft bodies 112A and 112B are each independently disposed in a rotatable condition. The bending operation knob 6 for left/right bending is fixedly installed at an upper portion of the shaft body 112A, and the sprocket 45 for left/right bending is fixedly installed at a lower portion thereof. On the other hand, on the shaft body 112B that is on the outer side of the shaft body 112A, the bending operation knob 4 for upward/downward bending is fixedly installed at an upper portion, and the sprocket 65 for upward/downward bending is fixedly installed at a lower portion thereof.

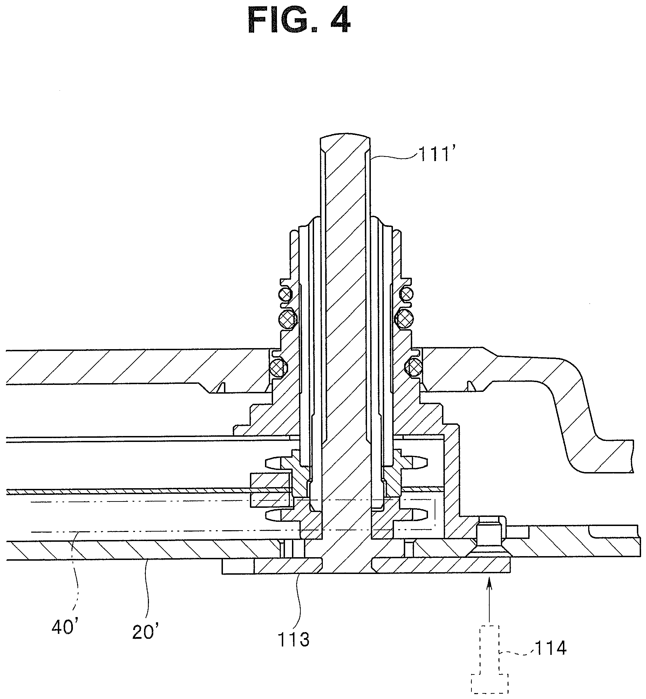

Conventionally, a fixing structure as shown in Fig. 4 is adopted for fixing the spindle 111 to the main frame 20. According to this conventional fixing structure, in a case of fixing a spindle 111' to a main frame 20', a flat-shaped connecting plate 113 is fixed by riveting to an end portion of the spindle 111', and thereafter the connecting plate 113 is fastened and fixed to the main frame 20' by means of a screw 114 as a fixing device.

In this case, in respect of achieving a reduction in weight and a reduction in cost, it is required for the main frame 20 (20') that is a built-in component of the operation portion 3 to be made the required minimum thickness, and it is also required for the connecting plate 113 to be made the minimum size. However, if the main frame 20' is made the required minimum thickness and the connecting plate 113 is made the minimum size and screwed and fixed thereto, there is a possibility that the distal end portion of the screw 114 will protrude from the main frame 20' and enter the movable range of the chain 40', and thus obstruct movement of the chain 40' and adversely influence bending operations.

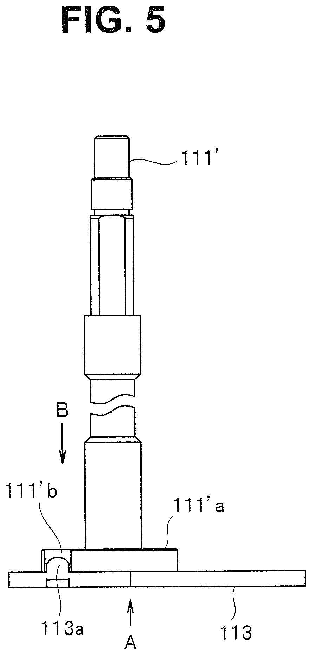

Consequently, in the conventional fixing structure a configuration is adopted in which the position of screwing and fixing the connecting plate 113 to the main frame 20' is set outside the movable range of the chain 40' so as not to obstruct movement of the chain 40'. However, in such case, the size of the respective members increases, and this is a factor that leads to an increase in weight and cost. Further, in the conventional fixing structure, for the purpose of positioning the spindle 111' and the connecting plate 113 with respect to the main frame 20', as shown in Fig. 5, a flange portion 111'a is provided at the shaft end of the spindle 111', and positioning in the circumferential direction is performed by fitting a protrusion 113a that is provided on the connecting plate 113 into a concave portion 111'b that is provided at a predetermined position of the flange portion 111'a.

Accordingly, when fixing the spindle 111' to the main frame 20', work is performed in which the axial direction positions of the spindle 111' and the connecting plate 113 are aligned from an A direction, next, the line of sight is inverted and the circumferential direction positions of the concave portion 111'b of the spindle 111' and the protrusion 113a of the connecting plate 113 are aligned from a B direction, and then, after returning the line of sight to the A direction and riveting the spindle 111' to the connecting plate 113, the connecting plate 113 to which the spindle 111' is fixed is fastened and fixed to the main frame 20' using the screw 114. In this fixing work, because the line-of-sight direction is inverted, it is necessary for the worker to invert the assembly parts or for the worker to alter their own posture to perform the assembly work, and consequently the working efficiency deteriorates.

In contrast, in the fixing structure of the spindle 111 and the main frame 20 in the present embodiment, a configuration is adopted that realizes a weight reduction and a cost reduction by eliminating the connecting plate 113 and reducing the size of members and also improves the working efficiency as a structure that enables simple fixing work. Specifically, returning to Fig. 3, in the present embodiment a structure is adopted in which a flange portion 111a is provided at the lower end of the shaft of the spindle 111, and the screw 114 is used to fasten and fix the flange portion 111a directly to an upper face side on which constituent members of the bending operation mechanism 100 of the main frame 20 are disposed as well as a lower surface side on the opposite side thereto.

A hole portion 111b through which the screw 114 is inserted for fastening and a hole portion 111c for positioning in the circumferential direction are provided in the flange portion 111a of the spindle 111 at positions that are adjacent to the outer circumferential face of the spindle 111. Further, an internal thread portion 20a in which the screw 114 is fastened is provided on a lower surface side of the main frame 20 with respect to the flange portion 111a of the spindle 111. A projecting portion 20b that fits into the hole portion 111c protrudes for positioning in the circumferential direction.

At this time, in the sprocket 45 that is disposed on the upper face of the main frame 20, a small-diameter portion 45b is formed by notching, in the circumferential direction, a distal end side of a protruding portion 45a that protrudes from the boss portion and contacts against the upper face of the main frame 20. A height Hj in the axial direction of the small-diameter portion 45b is set to a height such that the distal end portion of the screw 114 does not come in contact therewith. Further, the attachment position of the screw 114 is set so that the distal end portion of the screw 114 remains within a range D that is defined by an outer circumferential portion of the protruding portion 45a and a rotational trajectory of a connecting pin 40a of the chain 40.

By adopting this fixing structure between the spindle 111 and the main frame 20, while suppressing an increase in the diameter of the flange portion 111a, interference can be avoided between the distal end of the screw 114 protruding from the main frame 20, and the sprocket 45 and chain 40 for left/right bending that are disposed on the upper face of the main frame 20. As a result, elimination of the connecting plate 113 and a decrease in size of members can be realized, and reductions in weight and cost can be achieved.

Further, when performing work to fix the spindle 111 and the main frame 20, fixing by means of the screw 114 for positioning in the axial direction and positioning in the circumferential direction of the spindle 111 and the main frame 20 can be performed while viewing the components from the same direction. Accordingly, assembly work can be easily performed without the worker altering their posture during the work and without inverting the components, and thus the working efficiency can be improved.

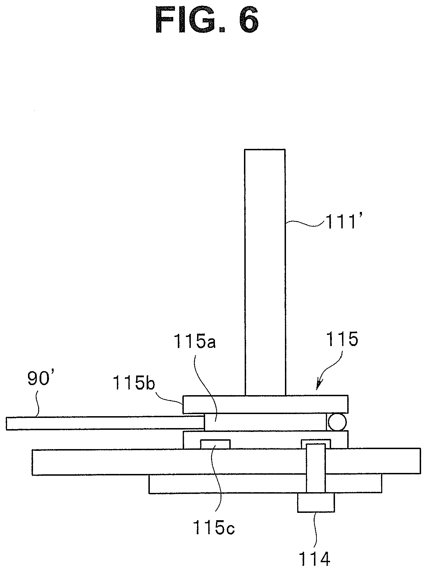

Note that the above described fixing structure between the spindle 111 and the main frame 20 can also be applied to a bending operation mechanism in which a chain is not used and instead a bending operation wire is wound around a pulley and is pulled to drive. As shown in Fig. 6, a pulley 115 for a bending operation which is disposed on the spindle 111" includes a winding portion 115a around which a wire 90' is wound, and a flange portion 115b that is vertically arranged on both sides of the winding portion 115a and serves as a guide for the wire 90'.

Since the flange portion 115b of the pulley 115 need only be provided at a portion which the wire 90' is wound around, a clearance portion 115c forming an annular concave portion in the axial direction of the spindle 111" can be provided on an inner diameter side of the winding portion 115a. The clearance portion 115c is formed so that the distal end portion of the screw 114 is accommodated therein when fastening and fixing a flange portion 111a" of the spindle 111" to a main frame 20" with the screw 114. By adopting such a fixing structure, it is possible to similarly achieve a weight reduction and improvement in the workability of the assembly work.

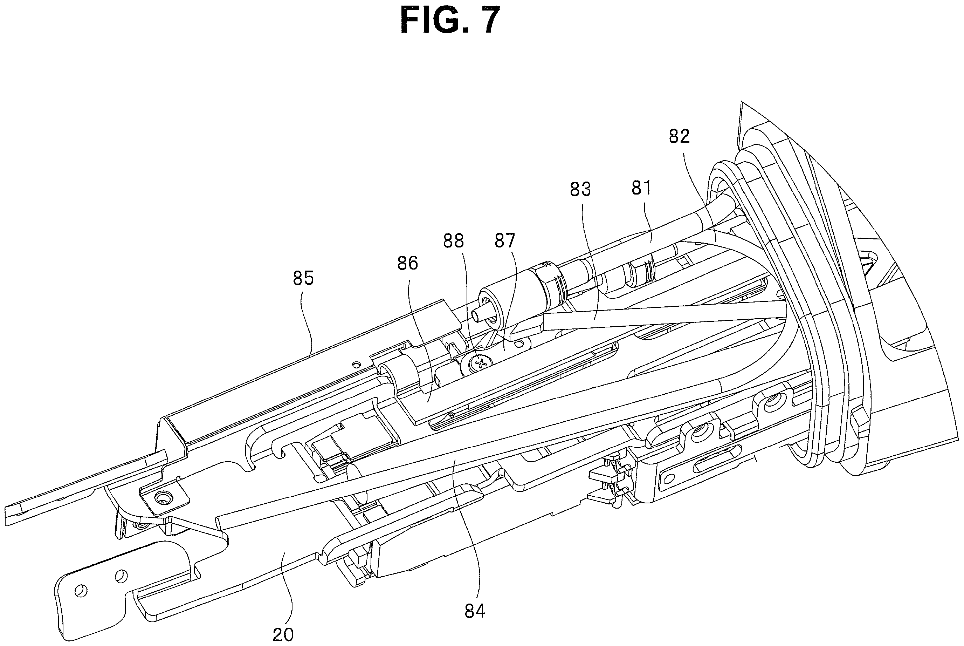

On the other hand, as shown in Fig. 7, various kinds of tubes and electrical cables, for example, a tube 81 that is used for purposes such as air/water feeding and suction, and electrical cables 82, 83 and 84 such as an image pickup cable that are extended from inside the universal cord 8 are built into the lower surface side of the main frame 20 on which the above described bending operation mechanism 100 is disposed. Further, various members such as a flexible substrate 86 that connects the switches 11 provided on the operation portion main body 3h and a control circuit board that is housed inside a substrate case 85 are also disposed on the lower surface side of the main frame 20.

These built-in members are compactly disposed, and because a tensile or compressive load is applied accompanying a bending operation of the insertion portion 2, it is necessary to secure space in which the respective members can move with respect to each other in order to lessen the influence of such a tensile or compressive load. However, when such a space is provided, there is a risk that a thin plate-like metal member such as an earth terminal 87 that connects the electrical cable 83 to GND will enter a gap between respective members, and will press against and cause damage to another built-in element when the tensile or compressive load is applied.

To deal with this situation, the earth terminal 87 is disposed at an inner position that avoids built-in members such as the tube 81, the electrical cable 82 and the flexible substrate 86, and is fastened and fixed to the main frame 20 using the screw 88. To fix the earth terminal 87 that is disposed on an inner side of such built-in members to the main frame 20 it is necessary to perform work to secure space in which to dispose the earth terminal 87 by pushing aside members with fingers of one hand or the like while paying attention to the tube 81, the electrical cable 82, the flexible substrate 86 and the like and to maintain that state, and then, with the other hand, dispose the earth terminal 87 at a predetermined position of the main frame 20 and fasten and fix the earth terminal 87 with the screw 88.

To tighten the screw 88 it is common to use an electric screwdriver to thereby shorten the assembly time. Conventionally, not only is the work to fix the earth terminal 87 complicated, but it also has not been easy to manually hold the earth terminal 87 during the work with an electric screwdriver. If the force holding the earth terminal 87 is insufficient, there is a risk that the earth terminal 87 will rotate together with the electric screwdriver and will damage peripheral members, and therefore the work to fix the earth terminal 87 using an electric screwdriver is work that requires extensive care.

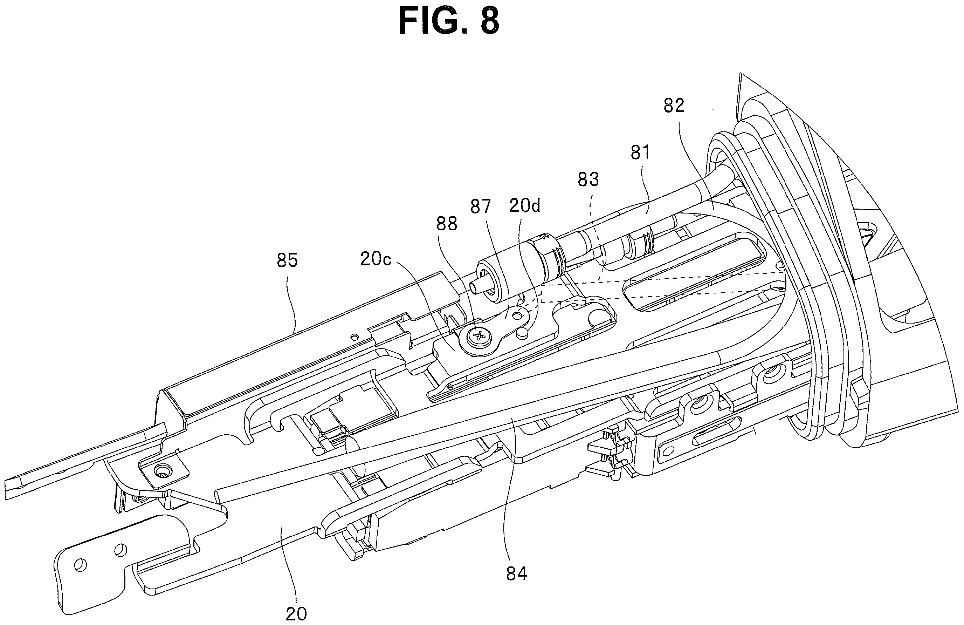

Therefore, as shown in Fig. 8, in the main frame 20, a terminal mounting portion 20c at which the earth terminal 87 is mounted has a bearing surface that is wider than the external shape of the earth terminal 87, and in the vicinity thereof a rotation stopping portion 20d is provided that is protruded in a convex shape or a wall shape as a rotation stopper that restricts rotational movement of the earth terminal 87. A protruding height of the rotation stopping portion 20d is formed to a height that is equal to or greater than the plate thickness of the earth terminal 87, and when fixing the earth terminal 87 to the main frame 20, work is performed to cause the side face of the earth terminal 87 to contact against the side face of the rotation stopping portion 20d of the main frame 20.

By this means, the earth terminal 87 can be easily held manually against the rotational torque of an electric screwdriver, and it is possible to perform the assembly work while positioning the earth terminal 87, and thus the assembly work can be made easy and fast. Further, because the height of the rotation stopping portion 20d is equal to or greater than the plate thickness of the earth terminal 87, built-in members disposed at the periphery thereof move at positions that are further upward than the earth terminal 87, and hence the earth terminal 87 does not enter and get caught in a gap between other members, and damage to other members can be prevented.

Note that, although an example in which the rotation stopping portion 20d serves as a rotation stopper when fastening the earth terminal 87 using the screw 88 is illustrated in Fig. 8, a configuration may also be adopted in which a rotation stopper is also provided in a direction for loosening a screw, and not just a direction for tightening a screw.

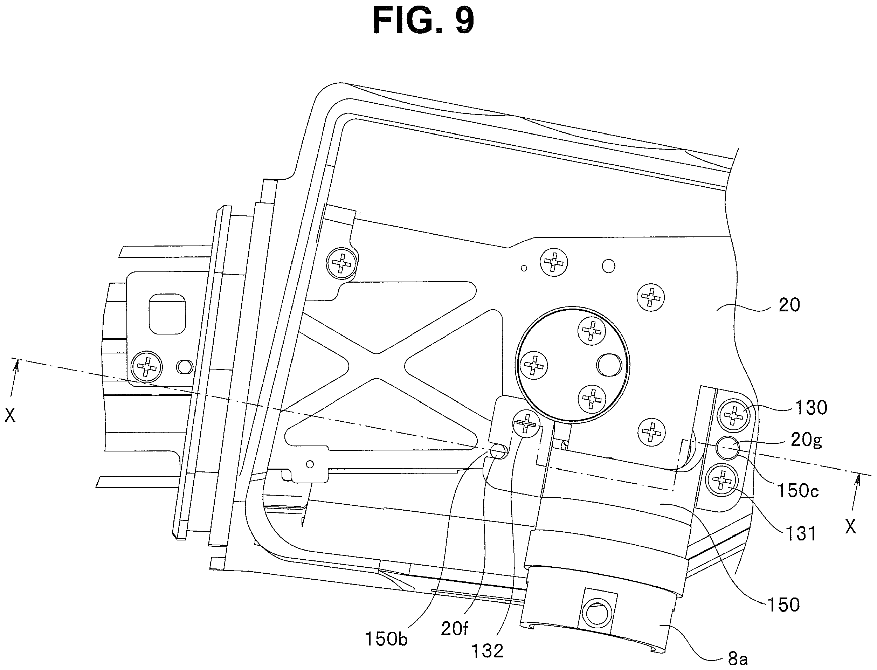

Next, the tube 81 and the electrical cables 82, 83 and 84 that are extended from inside the universal cord 8 which are disposed on the lower surface side of the main frame 20 are held by a cord fastening device 150 that is fixed to the main frame 20, as shown in Fig. 9, and are inserted into the operation portion main body 3h. The cord fastening device 150 is a gate-like member that connects the main frame 20 and a pipe sleeve 8a at a base portion of the universal cord 8, and is screwed and fixed thereto through a plurality of screws, in the present embodiment, screws 130, 131 and 132, on a hand side of the lower face of the main frame 20.

In this case, as described in the foregoing, to achieve a weight reduction and a cost reduction, the main frame 20 is formed with the required minimum thickness. Consequently, when the cord fastening device 150 is fastened and fixed using the screws 130 to 132, the distal end portions of the screws 130 to 132 protrude from the main frame 20 and there is a concern that interference may arise between the distal end portion of some of the screws and the lower-side chain cover 30. If a measure such as forming a hole in the lower-side chain cover 30 is taken to avoid interference with the lower-side chain cover 30, there is a risk that the chain 40 which slidingly moves over the lower-side chain cover 30 will catch at the edge of a hole or at a distal end portion of a screw that is protruding, thereby hindering movement of the chain 40.

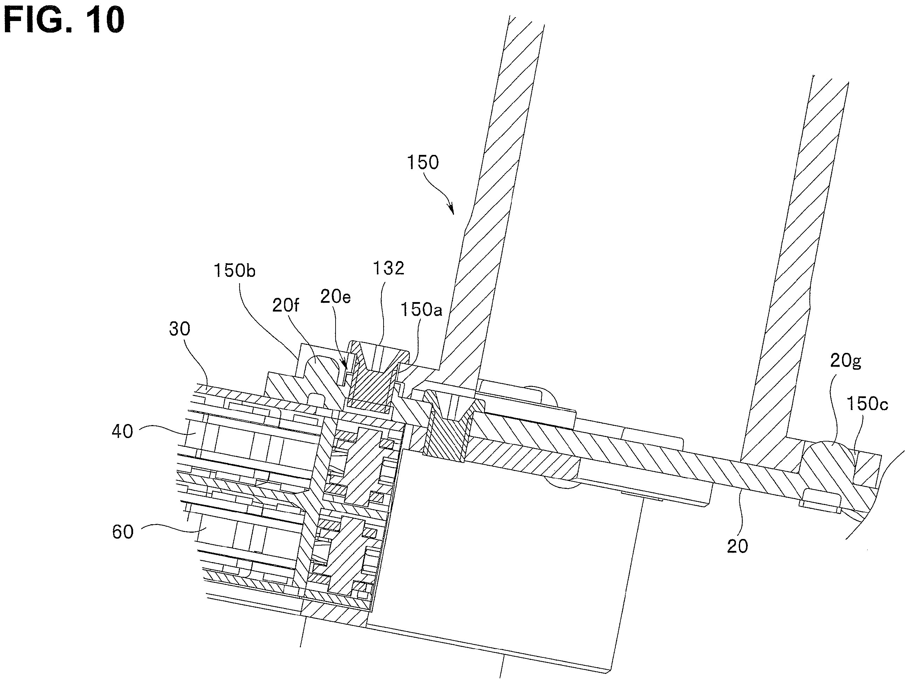

To deal with this situation, in the present embodiment, among the screws 130 to 132 that fix the cord fastening device 150 to the main frame 20, with respect to the screw 130 for which there is a risk of interfering with the lower-side chain cover 30, as shown in Fig. 10, a screw receiving portion 20e that protrudes from the lower surface side (cord fastening device 150 side) of the main frame 20 is provided at a corresponding position of the main frame 20, and a screw hole that forms an internal thread portion into which an external thread portion of the screw 130 is threaded is formed at the center of the screw receiving portion 20e. Further, in the cord fastening device 150, a concave portion 150a is formed in a direction away from the main frame 20, and the screw receiving portion 20e is housed in this concave portion 150a. An insertion hole through which the screw 130 is inserted is formed at the center of the concave portion 150a.

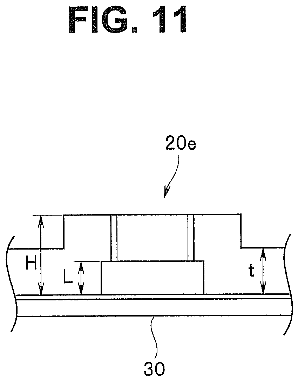

More specifically, the screw receiving portion 20e of the main frame 20 is not merely a cylindrical protruding portion in which an internal thread portion is provided that is formed by subjecting sheet metal to burring or the like, but rather is formed with a protrusion structure as shown in Fig. 11. That is, in the case of protrusion structures that are formed by burring, it is difficult to evenly control the protrusion heights, and the lengths of the internal thread portions vary. Therefore, by press working or the like of a sheet metal material, the screw receiving portion 20e according to the present embodiment is caused to protrude from the lower surface side (cord fastening device 150 side) of the main frame 20 and is formed in a cup-like stepped shape that is recessed from the upper face side (lower-side chain cover 30 side) to the lower surface side of the main frame 20, and is thus configured to ensure the dimensional accuracy of each portion.

By this means, the bottom portion of the cup shape in which the screw hole is formed can be evenly formed, the thickness of the bottom portion, that is, a difference (H-L) between a protruding height H of the screw receiving portion 20e and a depth L of the concave portion, can be formed so as to be equal to the plate thickness t of the main frame 20, and the required screw hole depth can be uniformly and accurately secured while maintaining the required minimum plate thickness. Further, relative to the underhead length of the screw 130 that is used, the depth L of the concave portion of the screw receiving portion 20e is formed to be a depth such that the distal end portion of the screw 130 does not protrude from the main frame 20, and thus interference between the distal end portion of the screw 130 and the lower-side chain cover 30 can be surely avoided.

Note that, at the time of assembly work to attach the cord fastening device 150 to the main frame 20, it is necessary to place the cord fastening device 150 on the main frame 20 at a deep position on the inner side of the operation portion main body 3h, and align the screwing positions and tighten the screws. In addition, at this time, since the state is one in which the end portion of the long universal cord 8 is not yet fixed, there is a risk that when fixing the cord fastening device 150 with screws, the cord fastening device 150 will be pressed by a built-in element such as an electrical cable or a tube and will become unstable, and the workability will decrease.

Consequently, when attaching the cord fastening device 150 to the main frame 20, the cord fastening device 150 is positioned by fitting convex portions 20f and 20g that are protruded from the main frame 20 into opening portions 150b and 150c of the cord fastening device 150. The convex portion 20g of the main frame 20 is fitted into the opening portion 150c of the cord fastening device 150 that is provided between the screw 130 and the screw 131, and the convex portion 20f of the main frame 20 is fitted into the opening portion 150b of the cord fastening device 150 that is provided in the vicinity of the attachment position of the screw 132.

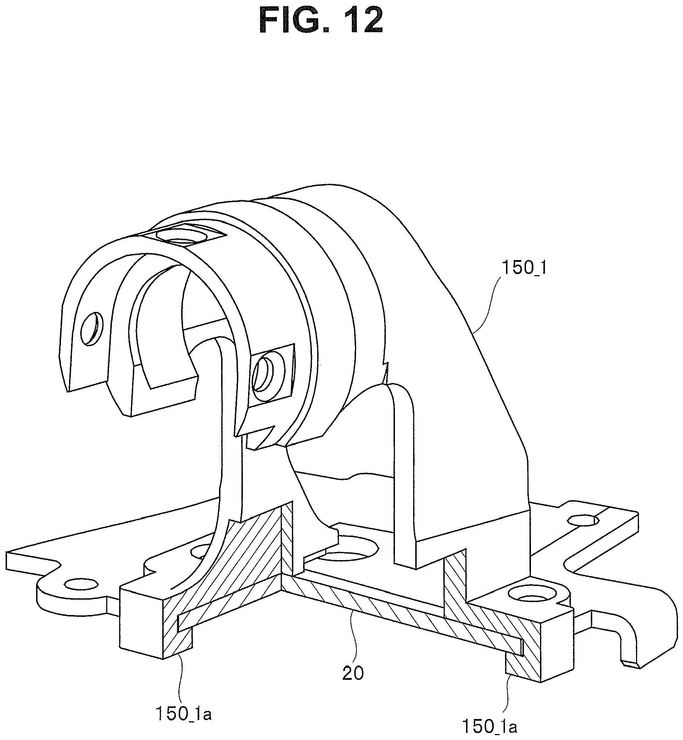

Further, to stabilize the work when fixing the cord fastening device 150 with screws, by employing a temporary fixing structure as exemplified in Fig. 12 to Fig. 14, the assembly workability can be improved. Note that, in the case of using the connecting plate 113 to fix the spindle of the main frame 20, it is also possible to apply these temporary fixing structures to the connecting plate 113.

A cord fastening device 150_1 with the temporary fixing structure exemplified in Fig. 12 is manufactured by a forming process, and not using sheet metal or cutting, and hooks 150_1a having a reversed "C" shape cross-section are provided that can be hooked in the thickness direction to the main frame 20 and temporarily fixed. The hooks 150_1a are formed from a plurality of directions such as the vertical and horizontal directions, and are assembled so as to insert a cord fastening device 150_1 onto the main frame 20.

Thus, the cord fastening device 150_1 can be temporarily fixed in a positioned state by assembling the cord fastening device 150_1 onto the main frame 20. Thereafter, it is sufficient to tighten the screws to permanently fix the cord fastening device 150_1. In this case, the above described temporary fixing is possible by only changing the shape of the cord fastening device 150_1, and without changing the main frame 20.

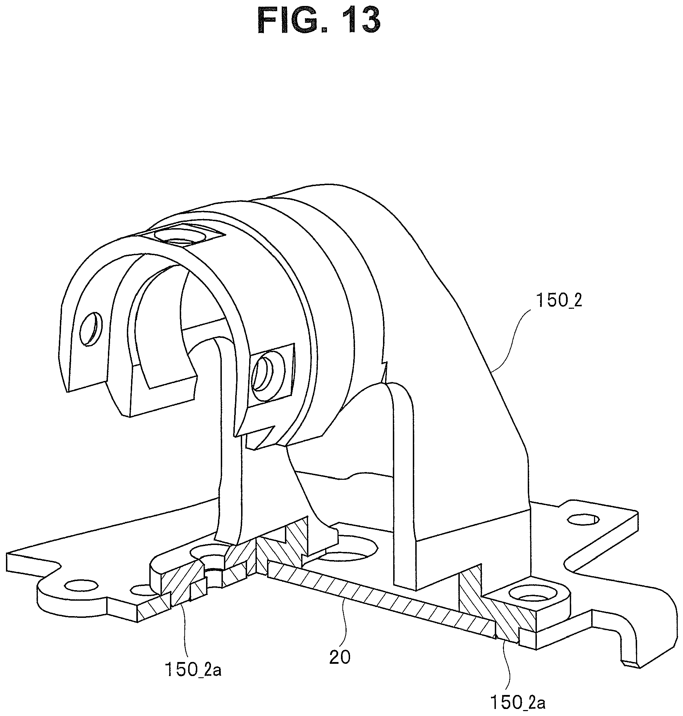

Further, a cord fastening device 150_2 exemplified in Fig. 13 is a device in which a cylindrical protrusion 150_2a is provided in the bottom face of the cord fastening device 150_2 by, similarly, a forming process. A hole is formed at a corresponding position of the main frame 20 to allow the protrusion 150_2a of the cord fastening device 150_2 to be inserted therein.

By this means, positioning and temporary fixing can be simultaneously performed by merely inserting the cord fastening device 150_2 from above the main frame 20. In this case, by molding the cord fastening device 150_2 using a resin material, it is possible to manually fit the cord fastening device 150_2 with a moderate resistance into the hole in the main frame 20, and thus the cord fastening device 150_2 can be temporarily fixed with greater ease. Thereafter, similarly to the foregoing cord fastening device 150_1, it is sufficient to tighten the screws to permanently fix the cord fastening device 150_2.

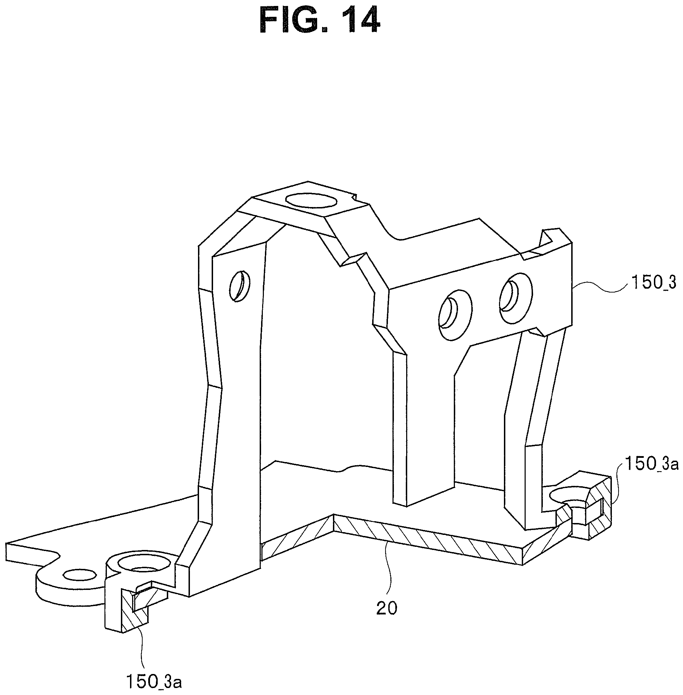

In addition, a cord fastening device 150_3 exemplified in Fig. 14 is a device in which one part of the conventional cord fastening device made of sheet metal is changed and a hook 150_3a that is similar to the hook 150_1a of the above described cord fastening device 150_1 is formed by bending. In the cord fastening device 150_3, in a case where the main frame 20 is not changed, the hook 150_3a can only be provided at a maximum of two places due to shape constraints.

In the case of the cord fastening device 150_3, although there are also cases in which firm positioning is difficult, temporary positioning can be performed with adequate strength for practical use, and the cord fastening device 150_3 can be adopted without changing the pipe sleeve 8a on the universal cord 8 side. In this case, if the shape of the main frame 20 is also changed, it is also possible to insert and position the cord fastening device 150_3 in a similar manner to the cord fastening device 150_1.

The present application claims priority from Japanese Patent Application No. 2014-041945 filed in Japan on March 4, 2014, the contents of which are hereby incorporated by reference in their entirety into the description, the claims and the drawings of the present application.

## Claims

1. An insertion instrument, comprising:
an insertion portion to be inserted into a subject;
an operation portion that is connected to the insertion portion and which an operator operates to actuate a distal end of the insertion portion;
a plate-like frame that is arranged inside the operation portion and that is fixed to the operation portion;
a long member that is arranged inside the operation portion and which actuates the distal end of the insertion portion in accordance with an operation of the operation portion;
a cover member which is arranged on the frame and that covers a portion in which the long member travels;
a fastening device that holds a member that is arranged inside the operation portion on an opposite side to the cover member of the frame;
a fixing device that fixes the fastening device to the frame; and
a receiving portion which is formed as a receiving portion for the fixing device in the frame, and which is protruded from a side facing the fastening device of the frame and is formed in a concave shape on a side facing the cover member.

2. The insertion instrument according to claim 1, wherein the fixing device is a screw having an external thread portion that is threaded into the receiving portion, and the receiving portion has an internal thread portion into which the external thread portion of the screw is threaded.

3. The insertion instrument according to claim 1 or 2, wherein the receiving portion maintains a plate thickness of the frame and is protruded on a side facing the fastening device, and is formed in a concave shape on a side facing the cover member.

4. The insertion instrument according to claim 1, wherein, in the fastening device, a concave portion is formed in a direction away from the frame, and the fixing device receiving portion is housed in the concave portion.

5. The insertion instrument according to claim 1, wherein the fastening device holds an electrical cable that is inserted through inside of the operation portion.

6. The insertion instrument according to claim 5, wherein a terminal mounting portion on which an earth terminal of the electrical cable is mounted, and a rotation stopping portion that restricts a rotational movement of the earth terminal are provided in the frame.
